(19) Europäisches Patentamt
European Patent Office
Office européen des brevets

(11) EP 0 564 466 B1

(12) **EUROPEAN PATENT SPECIFICATION**

(45) Date of publication and mention of the grant of the patent: **05.03.1997 Bulletin 1997/10**

(21) Application number: **91919435.7**

(22) Date of filing: **08.11.1991**

(51) Int Cl.[6]: **C07K 7/23**, A61K 38/24

(86) International application number: **PCT/EP91/02110**

(87) International publication number: **WO 92/08733 (29.05.1992 Gazette 1992/12)**

# (54) LHRH-ANTAGONISTS

LHRH-ANTAGONISTEN

ANTAGONISTES DE L'HORMONE DE LIBERATION DE LA LUTEOSTIMULINE (LHRH)

(84) Designated Contracting States:
**AT BE CH DE DK ES FR GB GR IT LI LU NL SE**

(30) Priority: **10.11.1990 CN 90108955**

(43) Date of publication of application: **13.10.1993 Bulletin 1993/41**

(73) Proprietor: **ASTA Medica Aktiengesellschaft D-01277 Dresden (DE)**

(72) Inventor: **XIAO, Shaobo, Tianjin Municipal Research Institute Nankai Dist, Tianjin 300191 (CN)**

(56) References cited:
**EP-A- 0 277 829**

- **ENDOCRINE REVIEWS, vol. 7, no. 1, The Endocrine Society; M.J. KARTEN et al., pp. 44-66**
- **ZEITSCHRIFT FÜR NATURFORSCHUNG, vol. 41c, 1986, Tübingen (DE); K. FOLKERS et al., pp. 1087-1091**
- **ZEITSCHRIFT FÜR NATURFORSCHUNG, vol. 42b, 1987, Tübingen (DE); K. Folkers et al., pp. 101-106**
- **R.W. ROESKE et al., "LhRh Antagonists With Low Histamine Releasing Activity", B.H. Vickery et al., (eds.), MTP Press, Boston, MD (US), 1987; pp. 17-24**
- **SCIENCE IN CHINA, vol. 34, no. 2, February 1991, series B; K.L. LIU et al., pp. 201-208**
- **INTERNATL. JOURNAL OF PEPTIDE & PROTEIN RESEARCH, vol. 35, no. 2, February 1990, Copenhagen (DK); K.L. LIU et al., pp. 157-160**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 564 466 B1

Printed by Jouve, 75001 PARIS (FR)

## Description

The present invention relates to novel peptides and their derivatives having exact chemical structure. The invention is also directed to the methods of their preparations and applications. Hypothalamic luteinizing hormone releasing hormone (LHRH) acts on the anterior pituitary gland to stimulate the secretion of luteinizing hormone (LH) and follicular stimulating hormone (FSH). The antagonistic analog of LHRH acts on anterior pituitary rapidly, lasts a long duration, can be safely and reversibly used for contraception or selectively suppression of gonadotropin secretion. For such kind of application, LHRH antagonists are superior to agonists. Up to now, there are more than two thousands of LHRH analogs have been designed and synthesized.

K. Liu et al., Int. J. Peptide Protein Res., 35 (2), pg. 157-160 (1990) disclose LHRH antagonists, wherein the amino acid in position 6 of LHRH is exchanged against derivates of phenylalanine.

M.J. Karten et al., Endocrine Reviews, 7 (1), pg. 44-66 (1986), describe GnRH analogs with modifications in position 2, 3, 6 or 10 and state that a mere increase in the number of amino acid substitutions doesn't necessarily lead to a higher potency.

K. Folkers et al., Z. Naturforsch., 41C, pg. 1087-1091 (1986), and ibid., 42B, pg. 101-106 (1987), teach certain modifications of LHRH.

R.W. Roeske et al., in B.H. Vickery and J.J. Nestor (Eds:), "LHRH Analogs: Contraceptive and Therapeutic Application", MTP Press, Boston 1987, pg. 17-24, disclose LHRH analogs with substitutions by MePal and /or Arg in position 5 and 6, respectively.

EP-A-0 277 829 describes LHRH analogs, wherein the arginine in Position 8 is alkylated on the side chain amino group.

The above citations show that only slight modifications in the protein sequence of LHRH lead to rather drastic changes in pharmacological properties.

"Nal-Arg" analog showed fairly high antifertility activity. However, "Nal-Arg" analog showed also very strong histamine-releasing activity (HRA). It caused transient edema of the face and extremities in rats when administrated at a dosage as high as 50-100 times of therapeutic dose. The result of clinical trial demonstrated histamine-related systemic effects. Other LHRH antagonists containing DArg$^6$ or DLys$^6$ showed similar side effects, their ED$_{50}$ for HRA were below 1 μg/ml. The present invention provides new LHRH antagonists which have very high antiovulatory activity (AOA) and very low histamine-releasing activity HRA) and negligible side effects. The contents and examples of this invention are as follows:

The design methodology of this invention is based on the topological similarity between the molecule of parent compound [NAc-D2Nal$^1$, DpClPhe$^2$, D3Pal$^3$, Ser$^4$, Tyr$^5$, DArg$^6$, Leu$^7$, Arg$^8$, Pro$^9$, DAla$^{10}$]NH$_2$ (II) and a neoropeptide, Substance P, which features the modification of both alkaline and lipophilic area in t molecular of the parent compound to give new antagonis having both high AOA and low HRA. The term "modification" hereof is adjusting or substitution of the amid acids in he area of Tyr$^5$-DArg$^6$-Arg$^8$ in C-terminus and the aromatic acids in N-terminus of (II More specifically, the design is introduction of suita alkaline group and substitutions of unnatural amino asids in position 2, 3, 5, 6, 8 of (II).

The following are also the methods and examples of this invention.

1. Substitution of D3pal which is a aromatic amino acid having suitabele basicity for DArg$^6$ in (II) t obtain analog (III): [NAc-D2Nal$^1$, DpClPhe$^2$, D3Pal$^3$ Ser$_4$, Tyr$_5$, D3Pal$_6$, Leu$_7$, Arg$_8$, Pro$_9$, DAla$_{10}$]NH$_2$

2. Substitution of Arg$^5$ for Tyr$^5$ in (III) to obtain (IV): [NAc-D2Na]$^1$, DpClPhe$^2$, D3Pal$^3$, Ser$^4$, Arg$^5$, D3Pal$^6$, Leu$^7$, Arg$^8$, Pro$^9$, DAla$^{10}$]NH$_2$

3. Substitution of Dphe$^3$ or its derivatives DXCH$_2$Phe for D3pal$^3$ in (IV) to obtain (V): [NAc-D2Nal$^1$, DpClPhe$^2$, DPhe$^3$, Ser$^4$, Arg$^5$, D3Pal$^6$, Leu$^7$, Arg$^8$, Pro$^9$, DAla$^{10}$]NH$_2$ or its (DXCH$_2$Phe$^3$) analogs.

4. Substitutions of Dphe$^3$ or its derivates for D3pal$^3$ in (III) to obtain (V'): [NAc-D2Nal$^1$, DpClPhe$^2$, DPhe$^3$, Ser$^4$, Tyr$^5$, D3Pal$^6$, Leu$^7$, Arg$^8$, Pro$^9$, DAla$^{10}$]NH$_2$ or its (DXCH$_2$Phe$^3$) analogs.

A series of new LHRH antagonists of the formula [NAc-D2Nal$^1$, AA$^2$, AA$^3$, Ser$^4$, AA$^5$, AA$^6$, Leu$^7$, AA$^8$, Pro$^9$ DAla$^{10}$] NH$_2$ have been synthesized, where AA are natura or unnatural amino acids which are expressed as D- or L-ArAla. More specifically herein,

AA$^2$ = D-pclPhe, D-ArAla, DPhe, Ar-Ala, DXCH$_2$Phe;
AA$^3$ = D3Pal, Ar-Ala, D-ArAla, DPhe, D-XCH$_2$Phe;
AA$^5$ = Arg, DMap, Pip, Tyr, Pal, Mop, Tep, Map, Phe, Eap, Pap, Bap, DMop;

$AA^6$ = D3Pal, D-Ar-Ala, D-$XCH^2$Phe;
$AA^8$ = Pip, Mop, Tep, Map, Eap, Pap, Bap, Arg;

in which

Ar =

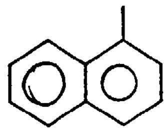 , 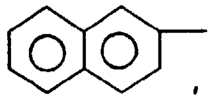 , H , 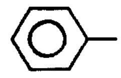 ,

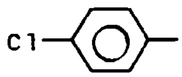 , 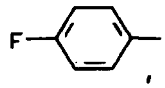 , 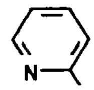 , 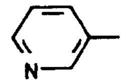 ,

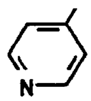 , 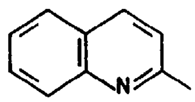 , 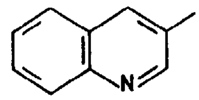 ,

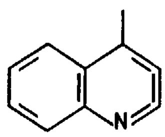 , 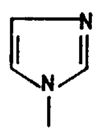 , 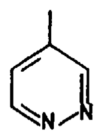 , 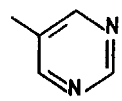 ,

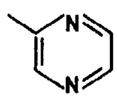 , 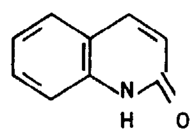

and

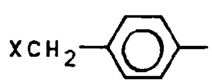 ,

in which

X=

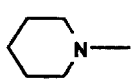 , 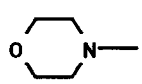 , 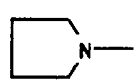 ,

and $R'_2N$-, RR'N-

in which

R' = $CH_3$-, $CH_3CH_2$-, $C_3H_7$-, $C_4H_9$-, H-;

R = $CH_3$-, $CH_3CH_2$-, $C_3H_7$-, $C_4H_9$-, H-;

The LHRH antagonists obtained by using the above described method, as a kind of peptide medicine, can be used to treat the disorder of reproductive endocrine system, such as edometriosis, precocious puberty of childeren and to treat prostate cancer and breast cancer as well as used as male or female contraceptives for birth control, or used in the diagnosis and treatment of intertility, etc. Such peptide medicine can be prepared as normal injection injectable capsules or other formulatations for real application.

Further description of this inventation is as follows:

In the natural course of histamine releasing in the body, neuropeptide substance P plays a very important role, its $ED_{50}$ for HRA is 5-15 μM. The chemical structure of SP is [$Arg^1$, $Pro^2$, $Lys^3$, $Pro^4$, $Gln^5$, $Gln^6$ $Phe^7$, $Phe^8$, $Gly^9$, $Leu^{10}$, $Met^{11}$]$NH_2$. The study on the relationship between its structure and HRA showed that $Arg^1$-$Pro^2$-$Lys^3$ in the N-terminus in the molecule of SP is essential for its HRA because deletion of these three amino acids from the molecule entirely abolished its HRA. By contrast, deletion of one two or three amino acids in C-terminus remained HRA as high as one fourth as HRA o itself. Further deletion of $Phe^8$ and $Phe^7$, HRA reduced 4 % and 0,57 % of those of SP. Further deletion of $Gln^{5.6}$ did not cause significant change of HRA. The above data implies that the liphophilic area around $phe^{7.8}$ determines the value of HRA, this area involves in the binding of molecule with the receptor of mast cell.

As mentioned previously, ($D2Nal^1$, $DArg^6$) analogs of LHRH showed very high HRA, its molecular structure has topological similarity with SP: $DArg^6$-$Leu^7$-$Arg^8$ in the former appears to be corresponding to $Arg^1$-$Pro^2$-$Lys^3$ in the latter, both consist of a pair of strongly basic amino acid residues between which only one neutral amino acid residue is present, i. e. both [$D2Nal^1$,DArg analog of LHRH and SP contains two strongly basic amino acid residues which are in 1,3 posititon relation ship. On the other hand, a clutter of aromatic amino acid residues in the former is considered to be corresponding to $Phe^{7.8}$ area in SP in terms of determination of he magnitude of HRA.

The design of this invention consists of two aspects: one is modifying $Tyr^5$-$DArg^6$-$Arg^8$ area in C-terminus, the other is fine adjusting the aromatic acids after optimizing the modificatin of the alkalious area in C-terminus. [NAc-$D2Nal^1$, $DpclPhe^2$, $D3Pal^3$, $Ser^4$, $Tyr^5$, $DArg^6$, $Leu^7$, $Arg^8$, $Pro^9$, $DAla^{10}$]$NH_2$ (II) is used as parent compund, which showed AOA 100 % at 0,5 μg in coin oil, 57 % at 0,25 μg.

First, $DArg^6$ in (II) could be replaced by weekly basic or neutral aromatic acids, such as D3Pal, D6Qal, tetrahydrotryptophan, methyl tryptophan. [NAc-$D2Nal^1$, $DpclPhe^2$, $D3Pal^{3.6.}$, $DAla^{10}$]LHRH(III) was optained when $D3Pal^6$ was substituted for $DArg^6$ in (II). (III) showed AOA 100 % at 3 μg, 83 % at 1 μg (in corn oil), and its $ED_{50}$ for HRA was 9.8 μg/ml, much better than that of "Nal-Arg" analog $ED_{50}$ for HRA was less than 1 μg/ml. It seems that the basicity of the whole molecule should equal to or closed to that of a pair of arginine in order to obtain high AOA. Because position 5 , like position 6, does not involve in the receptor binding, a wide variety of amino acid including arginine can be inserted in position 5. A series of new analogs were designed. For example, substitution of $Arg^5$ for $Tyr^5$ in (III) gave [NAc-$DNal^1$, $DpClPhe^2$, $D3Pal^{3.6.}$, $Arg^5$, $DAla^{10}$] LHRH (IV). Both (IV) and (II) contained two arginines, but the distance between two arginines in (IV), whose geometric relationship became 1, 4, i. e. there were two other amino acids between these two arginine, was larger than that in (II). Therefore, HRA would be reduced and, on the other hand, because of th presence of two arginine, AOA should not be lower than that of (II). The bioassay result of (IV) showed that $ED_{50}$ for HRA was 3,5 μg/ml, while AOA was 60 % at 0,12 μg (corn oil), 85 % at 0,25 μg, 100 % at 0,5 μg. This was the first time for LHRH antagonists to achieve $ED_{50}$ for AOA which was equal or less than 0,125 μg.

Therefore further design was based on the structure of (IV).

There are four alkalions residues, $D3Pal^{3,6}$ and $Arg^{5,8}$ in the molecule (IV), while (II) contains only three alkalinous. Therefore, it is resonable to replace one D3Pal by a neutral amino acid; on the other hand, (IV) showed very strong hydrophilicity and reducing the hydrophilicity by the substitution of a hydrophobic amino acid for DPal in (IV) would be beneficial to the retention of the drug in the body and then to the extension of the effective duration. A new series of analogs were then designed by substitution for $D3Pal^3$. (V) showed 100 % of AOA at 1 μg (in saline), equal to that of parent compund (IV), while HRA redued by a half: $ED_{50}$ for HRA was 7,4 μg/ml.

Further substitution of $DPhe^2$ for $DClPhe^2$ reduced the lipophilicity of this area in the molecule and reduced HRA.

$Arg^5$-$D3Pal^6$-$Leu^7$-$Arg^8$ in the C-terminus of (IV) seems to play a major role in triggering histamine releasing. D3Pal combines aromacity, basicity and hydrophilicity in one molecule, it is also stero-acceptable in LHRH antagonists for receptor binding. Similiarly, design of new series of unnatural amino acids prosessing the same character as D3Pal may lead to better LHRH antagonists that (IV) or (V).

Modification of natural, lipophilic, aromatic amino acid e. g. phenylalanine, for example, by means of the method described below in The Syntheis of Novel Unnatural Amino Acids, lead to a series of novel amino acids which combine aromacity, hydrophilicity and basicity in one melecule and can expressed the as formula: $R_1R_2NCH_2C_6H_4CH_2CH(NH)CO_2H$ (VI), were $R_1$ and $R_2$ may be the same or may differ each other, may be chain-like or cyclic, may also contain hetero-atom. With the $R_1$ and $R_2$ change, a series of amino acid can be obtained, which show systemically changed basicity, hydrophilicity and stero-character. Introduction of those amino acids in position 5, 6, 8 of (IV) have given three series of new antagonists of LHRH. The bioassay results showed that each series gave at least one new antagonist

showing 100 % AOA at 1 μg, similar to that of (IV), while HRA was significantly reduced. An example was (VII): [NAc-D2Nal$^1$, DpClphe$^2$, D3Pal$^3$, Ser$^4$, Mop$^5$, D3Pal$^6$, Leu$^7$, Arg$^8$, Pro$^9$, DAla$^{10}$]-$NH_2$, which showed 100 % of ADA at 1 μg, $ED_{50}$ 14,7 μg/ml for HRA, appeared better than (V). When substitution of (VI) for Arg$^8$ in (IV), the extent of HRA decreae was positively correlated to the length of R in (VI), so $ED_{50}$ for HRA could be higher than 200 μg/ml, such kind of compounds can be easily dissolved in aqueous solution and expected to be utilized clinically without formulation problems. The results demonstrated that Arg$^8$ or Lys$^8$ was not essential for highly potent LHRH antagonists. Suitable alkaline center in position 8 can ensure high AOA, meanwhile activity inducing mast cell to release histamine was remarkablely reduced when the basic enter mentioned above possesing significant stero-hinder.

This invention combining the modification in both N-and C-terminus lead to better LHRH antagonists.

The process of synthesis are illustrated as follows:

1. The synthesis of Novel Unnatural Amino Acids

Over 60 series and nonseries, D- or L-amino acids are designed and synthesized through the four synthetic routes outlined in the schema below. The structure of these unnatural amino acids are shown with the general structural formulas listed in the same schema. Some of these amino acids have alkalinity, hydrophilicity and aromaticity respectively, while the others have them all together in the molecule.

| Path 1 | Path 2 |
|---|---|

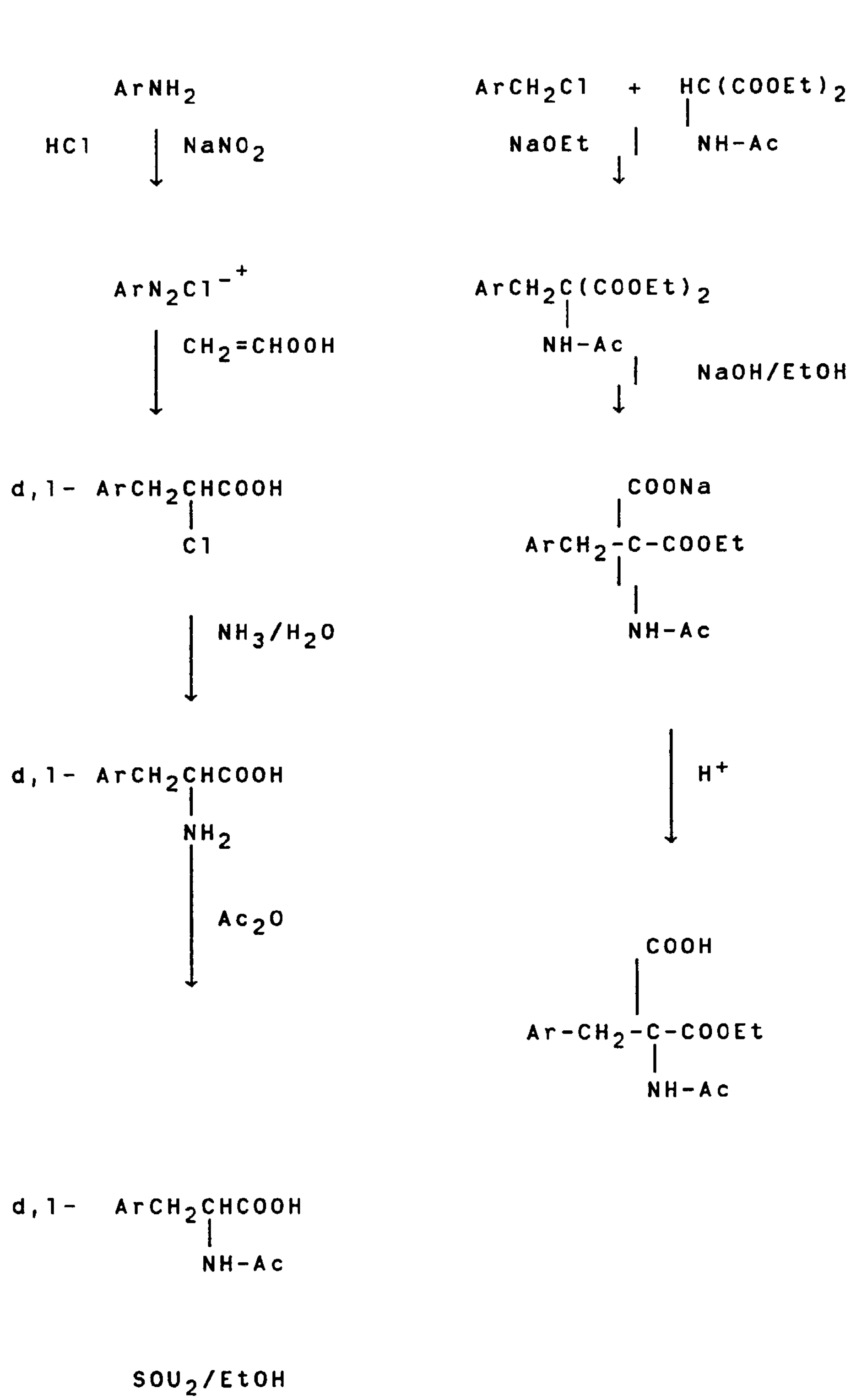

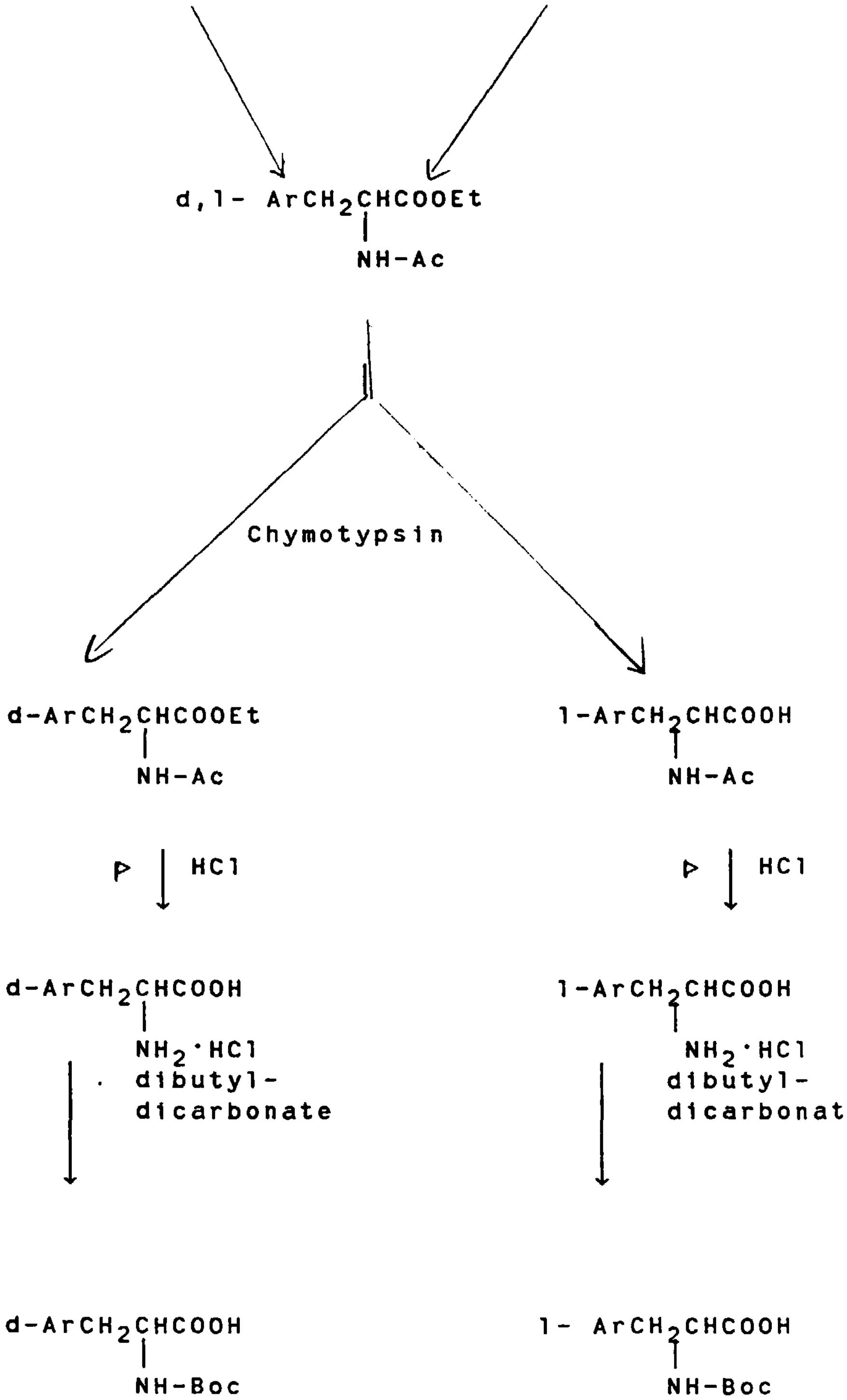
Chymotypsin
HCl
HCl
dibutyl-
dicarbonate
dibutyl-
dicarbonat

NH-Boc
|
Path I D or L-Ar-CH2-CH-COOH wherein

Ar = 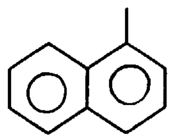 , 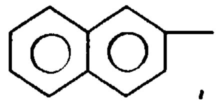 , , 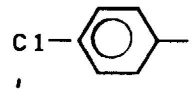 ,

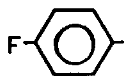

Path II D or L-ArCH2-CH-COOH wherein
|
NH-Boc

Ar = 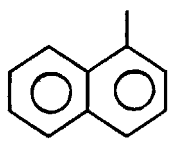 , 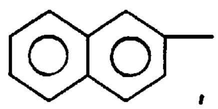 , , 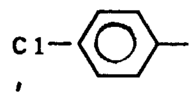 ,

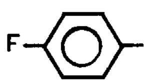 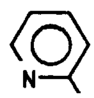 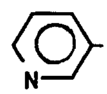

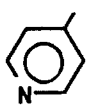 , 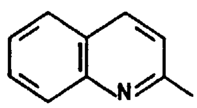 , 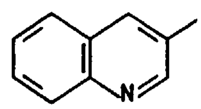 ,

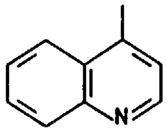,
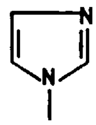,
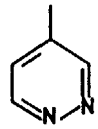,
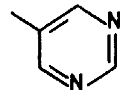,

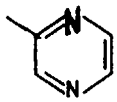

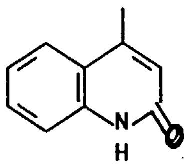,

and 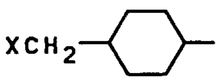,

X = 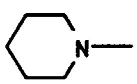, 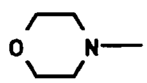, 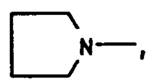, $R'_2N-$ , $RR'N-$

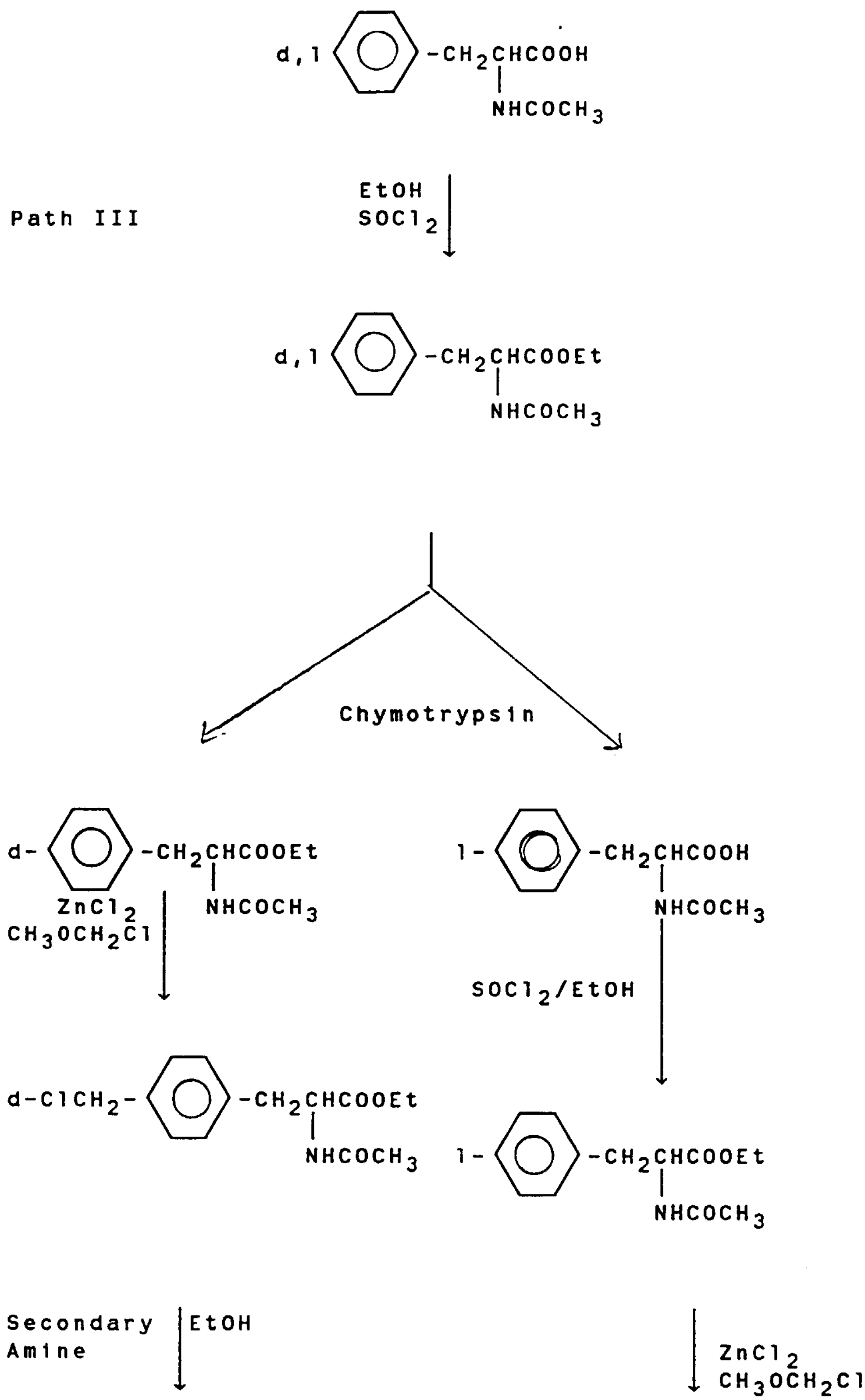
Path III
EtOH
SOCl2
Chymotrypsin
ZnCl2
CH3OCH2Cl
SOCl2/EtOH
Secondary Amine
EtOH
ZnCl2
CH3OCH2Cl

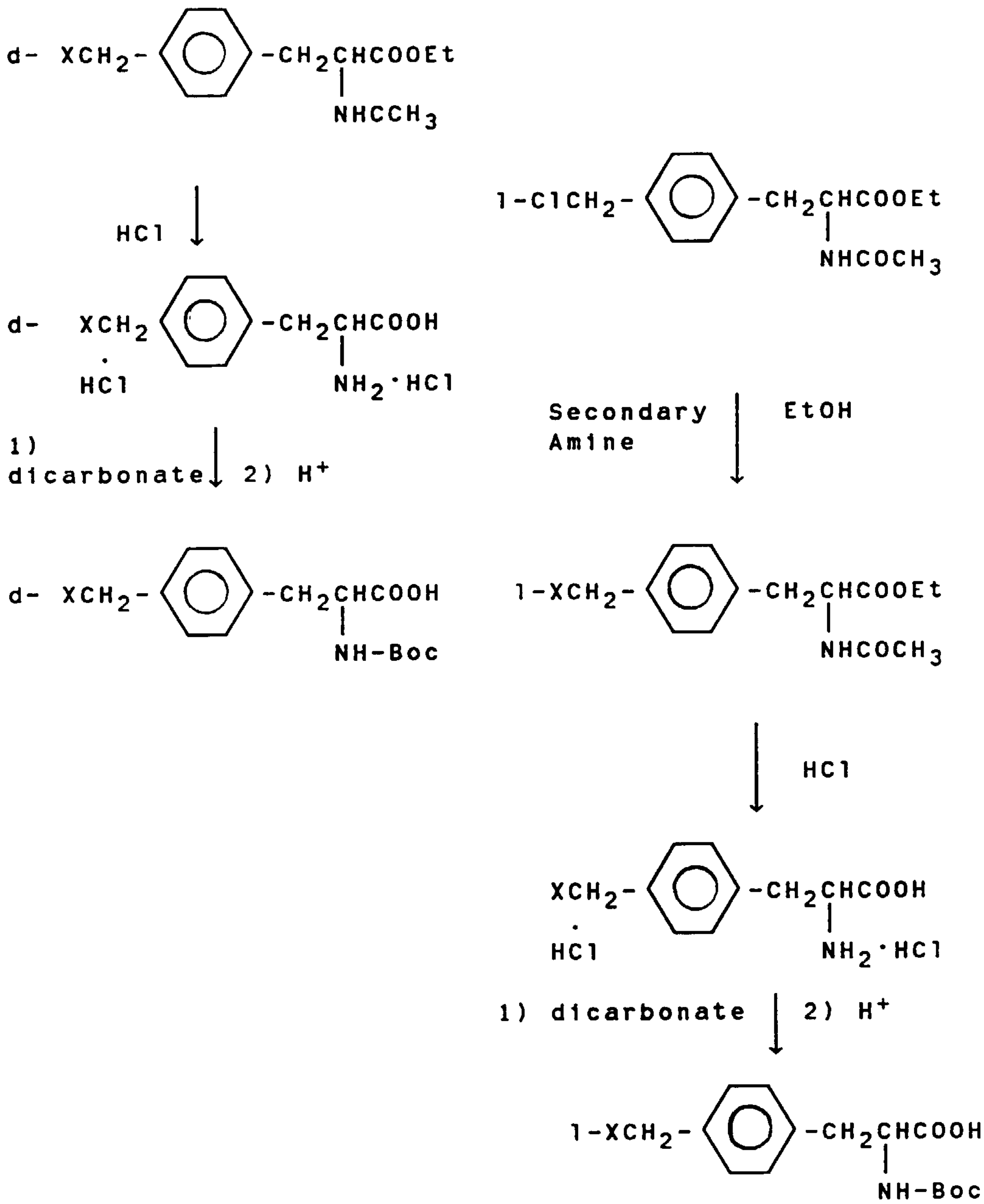


Path III

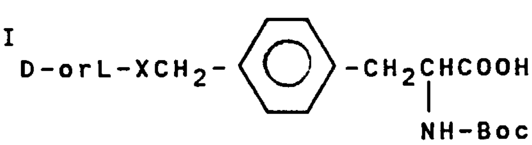

wherein

X= 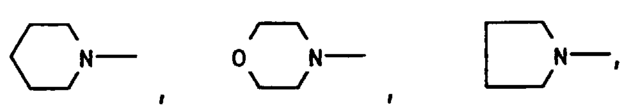, $R'_2N-$ , $RR'N-$

Path IV

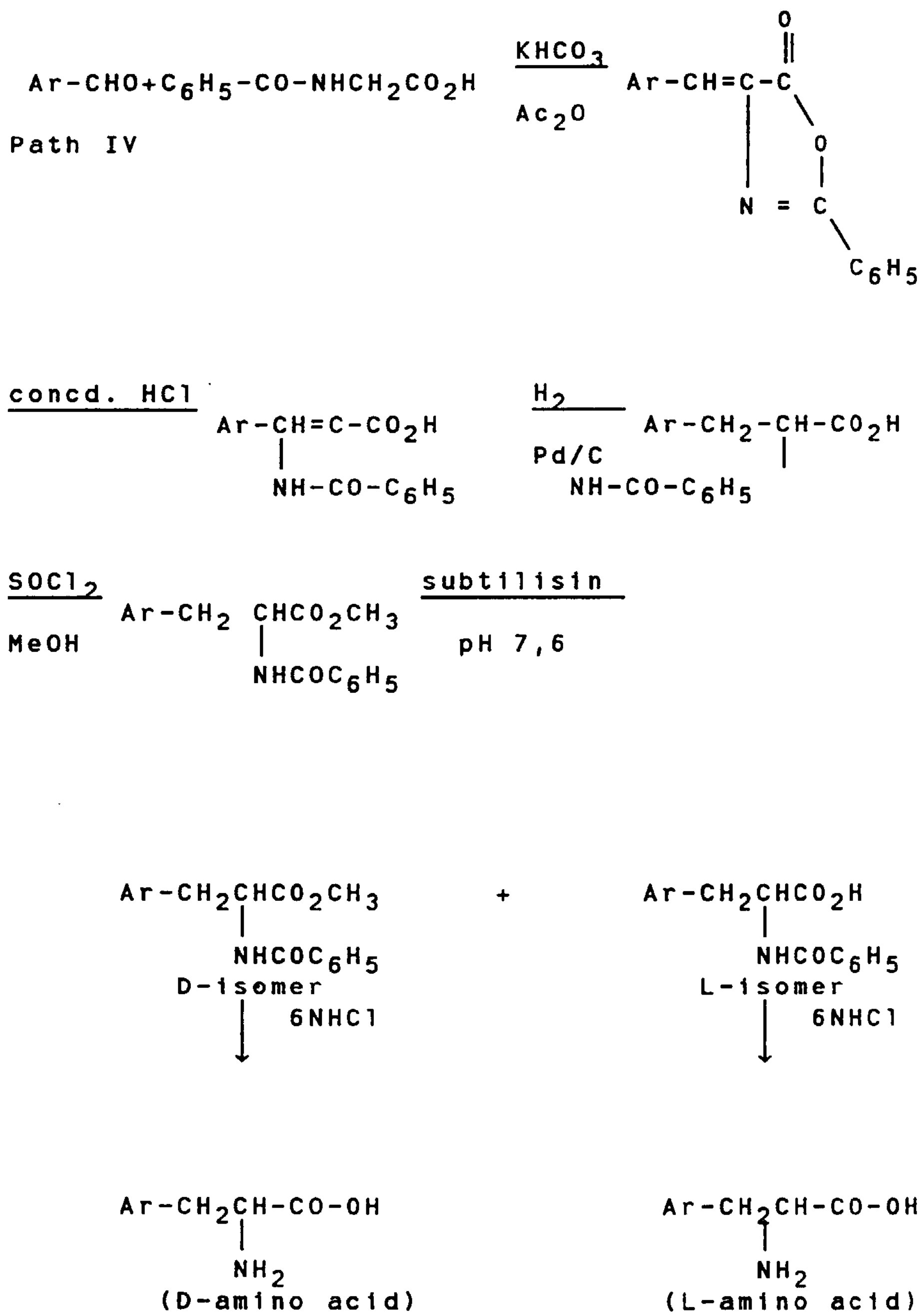
KHCO3
Ac2O
concd. HCl
H2
Pd/C
SOCl2
MeOH
subtilisin
pH 7,6
D-isomer
6NHCl
L-isomer
6NHCl
(D-amino acid)
(L-amino acid)

Path IV D or L 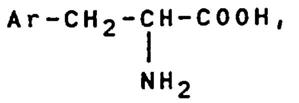 wherein

Ar = 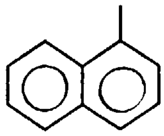 , 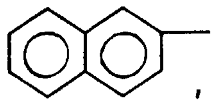 , ,

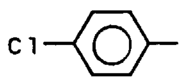 , 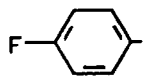 , 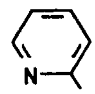 , 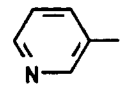 ,

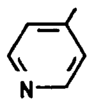 , 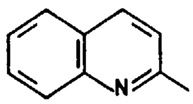 , 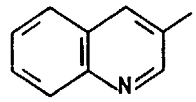 ,

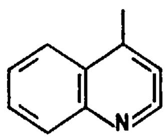 , 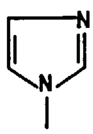 , 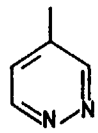 , 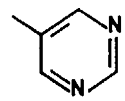 ,

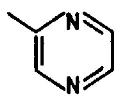 , 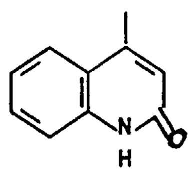

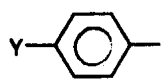 ,

Y = $(CH_3)_2N-$, $(CH_3CH_2)_2N-$, $(CH_3CH_2CH_2)_2N-$,

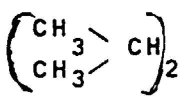 N-, $(CH^3CH^2CH^2CH^2)^2N-$

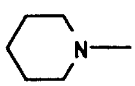 , 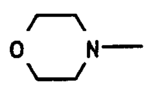 , 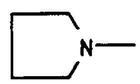 ,

2. Synthesis of Peptide

The synthesis begins from the C-terminus of the peptide on benzhydrylamine hydrochloride resin (BHA resin) utilizing the method of solid-phase peptide synthesis introduced by Merrifield. It is a three-step process including anchor, coupling and cleavage. Dichloromethane (DCM) is the major solvent used for washing between each step of reaction while isopropanol alcohol (IPA) and N,N-dimethylformamide (DMF) are also used when it is necessary. Catalyzed by excessive dicyclohexylcarbodiimide (DCC), coupling reaction is carried out, while adequate amount of 1-hydroxybenzotriazole (HOBT) is added. The degree of the coupling reaction is monitored with Kaises ninhydrin method. The second coupling reaction would be carried out if it gives a positive result in Kaises test. The peptide chain is cleaved from the resin using anhydrous hydrogen fluoride (HF) in the presence of anisole after the completion of all reactions necessary

on the resin, all of the temporary protecting group are deprotected at the same time. After washed by ethyl acetate or ether crude products of LHRH antagonists are obtained by aqueous acetic acid extraction followed by lyophilization. The yield is over 50 %.

3. Purification of Peptide

(1) The peptide is purified by gel permeation chromatography or silica partition chromatography through a column as high as 60 - 100 cm with the aid of UV/TLC monitoring. The LHRH antastits purified once are obtained after lyophilizing the major fractions. The yield is 50 - 90 % and the purity can be over 90 %.

(2) The peptide then is further purified on Waters high performance liquid chromatography (HPLC) instrument using reverse phase C18 column (7,8 x 300 mm) (μ-Bondapak 84176). The yield of this step is 20-50 % while the purity is no less than 99 %.

4. Purity Analysis of Peptide

(1) TLC analysis

It is carried out on a plastic sheet coated by silica gel 60 F254 of 5 - 10 cm height. They all shows a single spot when developed in five different solvent systems.

(2) HPLC analysis

They all shows a single peak when eluted with two kinds of solvent system, respectively, utilizing Waters HPLC instrument on a analytic column (μ-Bondapak 27324) when monitored by UV 210. The sample size is 10 - 200 μg.

5. Amino Acid Analysis of Peptide:

According to the PICO-TAG method developed by Waters Company, 50 μg of sample which have been dried under vacuum over 2 hours is weighed accurately on a $10^{-5}$g scale balance. After dissolved in water, 10 μg of aliquot is added to a reaction tube in which 1:1 hydrochloride acid (containing 1 % phenol) was added according to the manual.

The reaction lasts 22-24 hours at 105°C in a sealed container which had been filled with nitrogen and pumped to vacuum to remove the oxygen in reaction tube. Phenol isothiocyanate is added to derive the amino group after evaporating of excessive hydrochloride acid. Then it was analyzed with the HPLC-instrument equipped with PICO-TAG amino acid analytical column and monitored by UV254. The content of each amino acid and the relative mole ratio were calculated to give the amino acid composition of the sample based on the comparison of the integrated area of each amino acid to that of H-standard sample of Waters. The classical ion-exchange-ninhydrin derivation method (IEN) was also used as control which gave the same results. But it needed ten times more sample to get a satisfied result.

6. Evaluation of biological activity:

Corbin's rat antiovulatlon method is used. The healthy, adult, female SD rats (BW 200-250 g) are used in this experiment. All animals are maintained at 22-24 °C and on 14 h/10 h (light/dark) schedule. They are given standard food, and water ad libitum. The rats showing at least two consecutive 4-day estrous cycles in vaginal smear examination can be used in this experiment. The rats are given peptides (LHRH antagnists) at noon of proestrous with different dose in saline solution. The rats are sacrificed next day, their oviduct of two sides are examed under a dissecting microscope to determine the ovum number. The rats were divided into several groups according to the dosing, each group consists of about 10 rats, and the control group in which the rats are given equal amount of saline consists of 9-10 rats. The antiovulatory activity (AOA) is shown in the following equation:

$$AOA = \frac{\text{number of unovulated rats}}{\text{Total number of treated rats}} \times 100\ \%$$

7. Evaluation of Histamine Releasing Activity:

(1). Histamine releasing test (HRT) in vitro:

The healthy, adult, male SD rats (BW 200-250 g) housing in the above same conditions are used in this experiment. After anesthetized by $CO_2$ the peritoneal cavity is washed with 50 ml of PIPES AC medium containing 20 units of

heparin. Following centrifugation at 200xg for 8 min at 4 °C, cells are washed again and finally resuspended to a concentration of 8 to $24x10^5$ total leucocyted/ml in PIPAS AC. This suspension contains approximately 5-10 % mast cells. Washed cells are used immediately after collection and are prewarmed for 5 min at 37 °C prior to pipetting 0,3 ml aliquots into polystyrene tubes containing 0,3 ml of diluted peptide. The mixtures are incubated for 15 min at 37 °C and the reaction stopped by centrifugation at 400 xg for 15 min at 4 °C. The supernatans are assayed for histamine content by manual fluorometric assay method after successively extraction with n-butanol and n-heptane. The histamine content can be obtained from the histamine standard curve (see below). The percentage of histamine release can be calculted from the following equation:

$$\text{Histamine release (\%)} = \frac{\text{E-B}}{\text{C-B}} \text{ X } 100\ \%$$

where E is the fluorometric reading of experimental sample, B is the fluorometric reading of samples with cells and buffer only, and C ist the fluorometric reading of "complete" (cells treated with $HClO_4$).

The standard curve can be obtained by plotting the OD values on a fluorometer at 350 nm/450 nm (activation/ fluorescent) against the concentrations of serially diluted solution of accurately weighted histamine hydrochloride. The relative parameter r of the histamine standard curve can be 0.9998, and the lowest detectable concentration of histamine is 0,5 ng/ml.

The $ED_{50}$ value of peptide can be gotten from the dose response curves obtained by plotting the histamine release versus the peptide concentration on semilogarithmic paper.

All peptide samples should be tested with mast cells from a minimum of 3 different rats.

(2). Cutaneous anphylactoid activity test (CAT):

The healthy, adult, female SD rats (BW 250 g) are used in this experiment. The rats are injected intravenously with Evan's blue (1ml of 0,05 % solution). Immediately after that, the 0,05 ml of peptide solution (5, 0,5 and 0,05 µg/ ml respectively) and saline (control) are injected intradermally into a shaved section on the back of the animals. 30 minutes after the injection, the rats are sacrificed and the dorsal skin was reflected. The diameters of the lesions are measurd in millimeters in two perpendicular directions with a vernier caliper. The diameter of control is usually less than 5,5 mm.

The amount of Evan's blue permeating into the skin from the blood vessel can be spectrophometrically measured, too. The skin corresponding to the lesion area is cut down and immersed in a mixture of acetone/saline (7:3, Vol/Vol) overnight. After centrifugation next day, the content of Evan's blue in the supernatant is measured with a spectrophotometer (UV-260) at 610 nm against reference solutin of acetone/saline (7:3). Each peptide were tested in a minimum of 3 different rats.

A variety of new LHRH antagnists were designed and systhesized by means of the method described above. In brief, the new structure of LHRH antagonists was obtained by single or multiple substitution of the various natural and unnatural amino acids listed in the previous paragraphs.

A part of examples of new LHRH antagonists obtained thereupon are illustrated in table 1

Table 1: A Part of Examples Related to This Invention

| Analogue | AA 1 | AA 2 | AA 3 | AA4 | AA5 | AA 6 | AA7 | AA8 | AA9 | AA 10 |
|---|---|---|---|---|---|---|---|---|---|---|
| Parent | NAc-D2Na | DpClPhe | D3Pal | Ser | Tyr | DArg | Leu | Arg | Pro | DAla-NH2 |
| | | | | | Arg | | | | | |
| | | | | | Arg | DPhe | | Pip | | |
| | | | | | Arg | DMop | | Pip | | |
| | | | | | Arg | DPhe | | Mop | | |
| | | | | | Mop | D3Pal | | | | |
| | | | DPip | | Mop | DMop | | Pip | | |
| | | | | | Arg | D3Pal | | Pap | | |
| | | | | | Arg | D3Pal | | Pip | | |
| | | | | | DFPhe | D3Pal | | Pap | | |
| | | | | | | DPip | | Eap | | |
| | | | | | | DMap | | Mop | | |
| | | | DPhe | | Arg | DMop | | Map | | |
| | | | DPClPhe | | | DPip | | Map | | |
| | | | DPhe | | Arg | D3Pal | | | | |
| | | | | | Eap | | | Mop | | |
| | | | | | Tep | DMop | | Pep | | |
| | | | | | Tep | DMap | | Mop | | |
| | | | | | Tep | DEap | | | | |
| | | | | | Tep | DBap | | | | |
| | | | | | Tep | DPap | | | | |
| | | | | | Tep | DTep | | | | |
| | | DpFPhe | D3Pal | | | DMop | | Mop | | |
| | | DPhe | | | Mop | DMop | | Eap | | |
| | | | | | | DPip | | Pap | | |

Table 1: A Part of Examples Related to This Invention, Page 2

| Analogue | AA 1 | AA 2 | AA 3 | AA4 | AA5 | AA 6 | AA7 | AA8 | AA9 | AA 10 |
|---|---|---|---|---|---|---|---|---|---|---|
| Parent | NAc-D2Na | DpClPhe | D3Pal | Ser | Tyr | DArg | Leu | Arg | Pro | DAla-NH2 |
| | | | | | | DBap | | Pip | | |
| | | | | | Tep | DMop | | Eap | | |
| | | | | | | DMop | | | | |
| | | | | | | DTep | | | | |
| | | | | | Tep | DMop | | | | |
| | | | | | | DTep | | | | |
| | | DPClPhe | | | | | | | | |
| | | DPClPhe | | | | DMop | | Pip | | |
| | | | | | | DMop | | Bap | | |
| | | | | | Mop | | | | | |
| | | | | | Mop | D3Pal | | | | |
| | | | | | Arg | DMop | | | | |
| | | | | | Arg | DPlp | | | | |
| | | | | | Arg | DTep | | | | |
| | | | | | | D3Pal | | Pip | | |
| | | | | | Arg | D3Pal | | Pip | | |
| | | | | | Arg | D3Pal | | Mop | | |
| | | | | | Arg | D3Pal | | Tep | | |
| | | | | | Arg | D3Pal | | Pap | | |
| | | | | | Arg | DTep | | Pip | | |
| | | | | | Arg | DTep | | Mop | | |
| | | | DPhe | | Arg | DTep | | Tep | | |
| | | | DFPhe | | Arg | DTep | | Map | | |
| | | | DFPhe | | Arg | DTep | | Eap | | |
| | | | DFPhe | | Arg | DTep | | Pap | | |
| | | | Mep | | Tep | DPap | | Bap | | |

The Applications of This Invention:

1. After finishing the preclinical pharmacology and toxicology study, we can apply these new LHRH antagonists which have high therapeutic effectiveness and low side effect in clinic so as to develop new medicine for treating the endometriosis and their disorder in reproductive endocrine system including precious puperty of children, prostate cancer and breast cancer. Since they suppress the secretion of gonadotropin through competing receptor with endogenous LHRH, and act rapidly reversibly and safely, they can be further developed as new type of contraceptives for male or female. Besides, they can be also used in treatment of infertility and for selectively and reversibly abolishing the function of pitutary gland in terms of secreting gonadotropin.

Being a kind of peptide medicine, the LHRH antagonists desrcibed herein are unlikly to be administrated orally.

But they can be easily made into lyophilized powder which are ready to dissolve in saline for injecting iv, sc or im.

Morover, long-actin delivery systems, such as biodegradable, injectable capsules are studied. The capsules can be implanted subcutaneously by a special syringe and would be adsorbed by the tissue after release of all peptide contents and do not need to remove surgically. The long-acting delivery system is specially useful for long-term adminstration of LHRH analogues in clinic.

The following are the analyses results of the examples (taking three analogues IV, V, VII as typical examples):

(1) The Purity

Thin layer chromatography (TLC):

There is only a single spot in each of the chromatogram developed in five different solvent systems.

High performance liquid chromatography (HPLC):

There is only a single peak in each of the chromatogram eluted with two different sovent systems.

The values of Rf and retention time TR are shown in Table 2, also with reference of Figure 1-4.

Table 2:

| The chromatographic analysis results of LHRH antagonists | | | | | | | |
|---|---|---|---|---|---|---|---|
| Analogs | HPLC | | TLC | | | | |
| | TR1 | TR2 | Rf1 | Rf2 | RF3 | Rf4 | Rf5 |
| | | (min) | | | | | |
| IV<br>V<br>VII | 7.55<br>7.90<br>16.19 | 5.26<br>8.11<br>9.58 | 0.23<br>0,32<br>0.17 | 0.21<br>0.30<br>0.08 | 0.31<br>0.35<br>0.16 | 0.19<br>0.30<br>0.40 | 0.65<br>0.69<br>0.12 |

Solution A + 80 % acetonitrile

Solvent System 2:

Solution A is 0.01M $KH_2PO_4$ aqueous solution (pH3)

Solution B is 20 % solution A + 80 % acetonitrile

TLC solution system:

1. nBUOH/EtOAC/HOAC/$H_2O$ (5:5:1:1)
2. nBuOAC/nBuOH/HOAC/$H_2O$ (2:8:2:3)
3. nBuOAC/HOAC/$H_2O$ (4:1:5), up phase
4. nBUOH/HOAC/$H_2O$ (4:1:2)
5. nBUOH/EtOAC/HOAC/$H_2O$ (1:1:1:1)

(2) Amino acid analysis

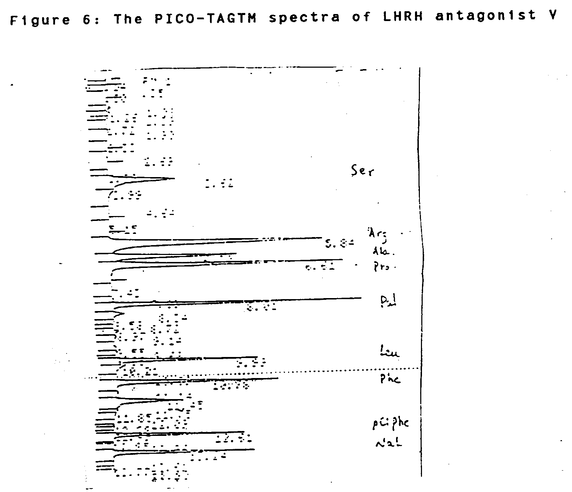

The analysis are carried out according to the method of classical IEN and new PIco-Tag, the results are shown in Table 3 and Figure 5,6.

Table 3:

| **The amino acid composition of LHRH antagonists** | | | | | | | | | | |
|---|---|---|---|---|---|---|---|---|---|---|
| Analogs | Methods | Ser | Arg | Ala | Pro | Leu | Phe | Pal | pCIPhe | Nal |
| IV | IEN | 0,86 | 2.05 | 1.01 | 0.99 | 1.13 | | + | + | ND |
| | Pico-TAG | 0,92 | 2.25 | 0.91 | 1.01 | 0,91 | | + | + | + |
| V | IEN | 0.81 | 2.02 | 1.03 | 1.03 | 0.12 | 0.99 | + | + | + |
| | Pico-TAG | 0.68 | 2.26 | 0.93 | 1.29 | 1.04 | 1.00 | + | + | + |
| VI | IEN | 0.91 | 0.91 | 1.00 | 1.00 | 1.09 | | + | + | ND |
| ND: Not determined | | | | | | | | | | |

(3) The bioassay results

The results of bioassays including antiovulatory activity at different doses and $ED_{50}$ for histamine-realising activity in vitro are illustrated in table 4 in which 26 antagonists are listed as examples.

Table 4:

| Bioassay Results of New LHRH Antagonists based on Parent structure | | | | | | | |
|---|---|---|---|---|---|---|---|
| | Substituted Amino Acids | % AOA/µg | | | | | HRA (µg/ml) |
| | | 0.125 | 0.25 | 0.5 | 1.0 | 2.0 | $ED_{50}$ ± SEM |
| 1 | Parent | | 50 | 75 | 100 | | 3.5 ± 0.38 |
| 2 | DPhe | | 29 | 60 | 100 | | 7.4 ± 0.98 |
| 3 | DPhe,DPhe | | | | 0 | | 18.5 ± 7.00 |
| 4 | DTyr, Lys | | | | 40 | | 5.1 ± 2.15 |
| 5 | D-Phe | | | | | 60 | 35.0 ± 5.05 |
| 6 | Map | | | 29 | | | 24.8 ± 4.47 |
| 7 | Eap | | | 43 | | | 12.0 ± 0.50 |
| 8 | Pap | | | 0 | | | 9.6 ± 0.19 |
| 9 | Bap | | | 14 | | | 23.5 ± 5.78 |
| 10 | D-Map | | | 12,5 | | | 18,3 ± 2,38 |
| 11 | Tep | | | 14 | | | 36.8 ± 5.68 |
| 12 | Pip | 17 | 33 | 71 | 100 | | 9.4 ± 1.63 |
| 13 | Mop | | | 25 | 100 | | 14.7 ± 2.70 |
| 14 | D-Map | | | 14 | | | 19.5 ± 2.50 |
| 15 | D-Eap | | | 14 | | | 13.0 ± 1.00 |
| 16 | D-Tep | | | 71 | | | 22.5 ± 3.25 |
| 17 | D-Pip | | | 0 | 50 | 57 | 7.6 ± 2.48 |
| 18 | D-Mop | | 33 | 67 | 100 | | > 11 |
| 19 | Map | | | 57 | 100 | | 5.4 ± 1.22 |
| 20 | Eap | | | | 29 | | 56.9 ± 15.1 |
| 21 | Pap | | | | 50 | 88 | 70.4 ± 26.8 |
| 22 | Bap | | | | 0 | | > 235 |
| 23 | Tep | | | | 100 | | 6.6 ± 2.13 |
| 24 | Pip | | | | 43 | | 27.5 ± 2.50 |
| 25 | Mop | | | | 71 | | 52.5 ± 17.5 |
| 26 | D-Map | | | | 0 | | 28.0 ± 9.00 |
| * The parent structure is: [NAc-D2Nal$^{1}$, DpClphe$^{2}$, D3Pal$^{3}$, Ser$^{4}$, Arg$^{5}$, D3Pal$^{6}$, Leu$^{7}$, Arg$^{8}$, Pro$^{9}$, DAla$^{10}$]$NH_2$ | | | | | | | |

As illustrated and described above, the LHRH antagonists designed and synthesized according to this invention shows very good properties. They are pure in TLC or HPLC analysis. They are pure in TLC or HPLC analysis. Their compositions are correct, i. e., the same is designed. Their antifertile activity is high: they can inhibit rat ovulation when injected s. c. at the dosage of 0.1 to 2.0 µg at the noon of proestrus. Their histamine related side effect is low: their $ED_{50}$ for in vitro histamine releasing activity (the effective dose for rat mast cell to release 50 % of histamine) is ranged 5-300 µg/ml; the lession induced by them in the cutaneous anaphylactoid test in rats is as small as required in clinic. Their warer-solubility is very good, all bioasays are carrid our in saline solution, so they are easy to formulated for injection in clinic. They are also ready to formulated as long-acting delivery systems, among which injectable microcapsules are most convenient for long-term suppression of gonadotropin and gonadal hormone. Therefore, they can be used as highly effective, reversible and safe contraceptives for both male and female. They can be also utilized for treatment of various diseases related to disorders of reproductive endocrine such as hormonedependent prostate cancer and breast cancer, endometriosis, precosious puberty of children. They are also useful in treatment of infertility. The new LHRH antagonists herein can also be utilized in the basic research of reproductive physiology and pharmacology, such as in the study on the function of piturtary gland, on the effect of gonadal hormones or gonadotrpins or LHRH on sexual behaviour, etc.

ABBREVIATIONS

The following are abbreviations which have been used in the text of this patent application document.

| | |
|---|---|
| Ala | alanine |
| AOA | antiovulatory activity |
| Arg | arginine |
| Bap | dibutylaminomethyl phenylalanine |
| Boc | t-butyloxylcarbonyl |
| BuOAC | butyl acetate |
| CAT | cutaneous anaphalactoid test |
| DCC | dicyclohexylcarbodiimide |
| DCM | dichloromethane |
| D2Nal | D-ß-(2-naphthyl) alanine |
| D3Pal | D-ß-(3-pyridyl) alanine |
| DpClPhe | p-chloro-D-phenylalanine |
| DpFPhe | p-fluoro-D-phenylalanine |
| D6Qal | D-ß-(6-quinolyl) alanine |
| DMF | N,N-dimethyl formamide |
| Eap | diethylaminomethyl phenylalanine |
| $ED_{50}$ | effective dose for 50 % response |
| EtOAC | ethyl acetate |
| FSH | follicle-stimulating hormone |
| Glu | glutamic acid |
| Gly | glycine |
| His | histidine |
| HOBT | 1-hydroxybenzoltriazole |
| HPLC | high performance liquid chromatography |
| | ninhydrin derivation |
| HRA | histamine-releasing activity |
| HRT | histamine-releasing test |
| IEN | ion exchange chromatography with post-column |

| | |
|---|---|
| IPA | isopropyl alcohol |
| LH | luteinizing hormone |
| LHRH | luteinizing hormone releasing hormone |
| Leu | leucine |
| Lys | lysine |
| Map | dimethylaminomethyl phenylalanine |
| Met | methionine |
| Mop | mophorlinomethyl phenylalanine |
| nBuOH | n-butyl alcohol |
| NS | normal saline |
| Pap | dipropylaminomethyl phenylalanine |
| Phe | phenylalanine |
| Pip | piperidinomethyl phenylalanine |
| Pipes | piperazine-N,N'-bis[2-ethanesulfonic acid] |
| Pro | proline |
| Rf | rate of flow |
| SE | standard error |
| Ser | serine |
| TFA | trifluoracetic acid |
| TLC | thin-layer chromatography |
| TR | retention time |
| Trp | tryptophan |
| Tyr | tyrosine |
| Tep | tetrahydroperrolyl methyl phenylalanine |

## Claims

1. [Nac-D2Nal$^1$, DpClPhe$^2$, Dphe$^3$, Ser$^4$, Arg$^5$, D3Pal$^6$, Leu$^7$, Arg$^8$, Pro$^9$, DAla$^{10}$]$NH_2$

2. [NAc-D2Nal$^1$, DpClPhe$^2$, DPhe$^3$, Ser$^4$, Tyr$^5$, D3Pal$^6$, Leu$^7$, Arg$^8$, Pro$^9$, DAla$^{10}$]$NH_2$

3. [N-AC-D2Nal$^1$, P-Cl-D-Phe$^2$, D3Pal$^3$, Ser$^4$, Mop$^5$, D3Pal$^6$, Leu$^7$, Arg$^8$, Pro$^9$, D-Ala$^{10}$]$NH_2$

4. [N-AC-D-2Nal$^1$, D-Phe$^2$, D3Pal$^3$, Ser$^4$, Mop$^5$, D3Pal$^6$, Leu$^7$, Arg$^8$, Pro$^9$, D-Ala$^{10}$]$NH_2$

5. [N-AC-D-2Nal$^1$, P-Cl-D-Phe$^2$, D3Pal$^3$, Ser$^4$, Arg$^5$, D3Pal$^6$, Leu$^7$, Pap$^8$, Pro$^9$, D-Ala$^{10}$]$NH_2$

6. The use of one of the LHRH antagonists of claims 1 to 5 for the production of a medicament for the treatment of disorders of the reproductive endocrine system and prostate and breast cancer.

7. The use of one of the LHRH antagonists of claims 1 to 5 for the production of a medicament for the use as contraceptive as well for diagnosis and treatment of infertility.

8. A method for producing the LHRH antagonists of claims 1 to 5 wherein the alkalinous and lipophilic areas of [NAC-D2Nal$^1$, DpClPhe$^2$, D3Pal$^3$, Ser$^4$, Tyr$^5$, Arg$^6$, Leu$^7$, Arg$^8$, Pro$^9$, DAla$^{10}$]NH2 are modified by the replacement of the respective amino acids.

**Patentansprüche**

1. [NAC-D2Nal$^1$, DpClPhe$^2$, Dphe$^3$, Ser$^4$, Arg$^5$, D3Pal$^6$, Leu$^7$, Arg$^8$, Pro$^9$, DAla$^{10}$]$NH_2$

2. [NAC-D2Nal$^1$, DpClPhe$^2$, DPhe$^3$, Ser$^4$, Tyr$^5$, D3Pal$^6$, Leu$^7$, Arg$^8$, Pro$^9$, DAla$^{10}$]$NH_2$

3. [NAC-D2Nal$^1$, P-Cl-D-Phe$^2$, D3Pal$^3$, Ser$^4$, Mop$^5$, D3Pal$^6$, Leu$^7$, Arg$^8$, Pro$^9$, D-Ala$^{10}$]$NH_2$

4. [NAC-D-2Nal$^1$, D-Phe$^2$, D3Pal$^3$, Ser$^4$, Mop$^5$, D3Pal$^6$, Leu$^7$, Arg$^8$, Pro$^9$, D-Ala$^{10}$]$NH_2$

5. [NAC-D-2Nal$^1$, P-Cl-D-Phe$^2$, D3Pal$^3$, Ser$^4$, Arg$^5$, D3Pal$^6$, Leu$^7$, Pap$^8$, Pro$^9$, D-Ala$^{10}$]$NH_2$

6. Der Gebrauch von einem der LHRH-Antagonisten nach den Ansprüchen 1 bis 5 zur Herstellung eines Medikaments für die Behandlung von Störungen des endokrinen Fortpflanzungssystems sowie von Prostata- und Brustkrebs.

7. Der Gebrauch von einem der LHRH-Antagonisten nach den Ansprüchen 1 bis 5 zur Herstellung'eines Medikaments zur Verwendung als Empfängnisverhütungsmittel sowie als Mittel zum Diagnostizieren und Behandeln der Unfruchtbarkeit.

8. Ein Verfahren zur Herstellung der LHRH-Antagonisten nach den Ansprüchen 1 bis 5, bei dem die alkalinischen und lipophilen Bereiche von
[NAc-D2Nal$^1$, DpClPhe$^2$, D3Pal$^3$, Ser$^4$, Tyr$^5$, Arg$^6$, Leu$^7$, Arg$^8$, Pro$^9$, DAla$^{10}$]NH2
durch Substituierung der entsprechenden Aminosäuren modifiziert sind.

**Revendications**

1. [Nac-D2Nal$^1$, DpClPhe$^2$, Dphe$^3$, Ser$^4$, Arg$^5$, D3Pal$^6$, Leu$^7$, Arg$^8$, Pro$^9$, DAla$^{10}$]$NH_2$.

2. [Nac-D2Nal$^1$, DpClPhe$^2$, DPhe$^3$, Ser$^4$, Tyr$^5$, D3Pal$^6$, Leu$^7$, Arg$^8$, Pro$^9$, DAla$^{10}$]$NH_2$

3. [N-AC-D2Nal$^1$, P-Cl-D-Phe$^2$, D3Pal$^3$, Ser$^4$, Mop$^5$, D3Pal$^6$, Leu$^7$, Arg$^8$, Pro$^9$, D-Ala$^{10}{}_2$]$NH_2$

4. [N-AC-D-2Nal$^1$, D-Phe$^2$, D3Pal$^3$, Ser$^4$, Mop$^5$, D3Pal$^6$, Leu$^7$, Arg$^8$, Pro$^9$, D-Ala$^{10}$]$NH_2$.

5. [N-AC-D-2Nal$^1$, P-Cl-D-Phe$^2$, D3Pal$^3$, Ser$^4$, Arg$^5$, D3Pal$^6$, Leu$^7$, Pap$^8$, Pro$^9$, D-Ala$^{10}$]$NH_2$

6. Utilisation d'un des antagonistes de LHRH selon les revendications 1 à 5 pour la production d'un médicament pour le traitement des troubles du système endocrine de la reproduction et pour le traitement du cancer de la prostate et du sein.

7. Utilisation d'un des antagonistes de LHRH selon les revendications 1 à 5 pour la production d'un médicament destiné à l'emploi comme contraceptif ainsi que pour le diagnostic et le traitement de la non fertilité.

8. Un procédé pour produire les antagonistes de LHRH selon les revendications 1 à 5,
caractérisé en ce que
les zones alcalines et lipophiles de
[Nac-D2Nal$^1$, DpClPhe$^2$, D3Pal$^3$, Ser$^4$, Tyr$^5$, Arg$^6$, Leu$^7$, Arg$^8$, Pro$^9$, DAla$^{10}$]$NH_2$
sont modifiées par le remplacement des aminoacides respectifs.

Figure 1: The TLC result of LHRH antagonists IV, V, VII in five different systems

Sample IV

Sample V

Sample VII

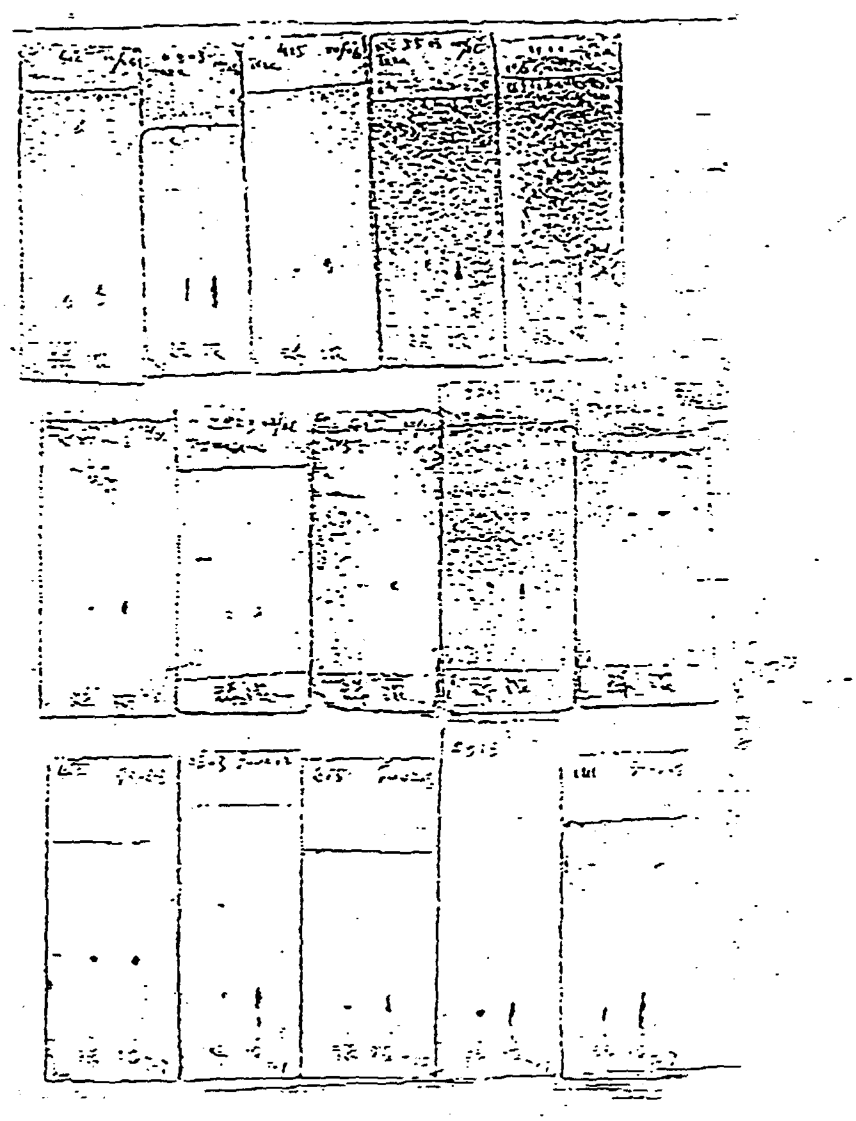

Figure 2: The reversed phase HPLC spectra for the pure sample of LHRH antagonist IV

Conditions:

Column: μ-Bondapak C18 (3.9 mm X cm)

moble phase: A, 0,1 M $NH_4OAC$ (pH7)

B, 20 % A + 80 % acetonitrile

gradient procedure: B from 10 % to 100 % in 15 minutes

flow rate: 2 ml/minute

detector: UV 229 nm

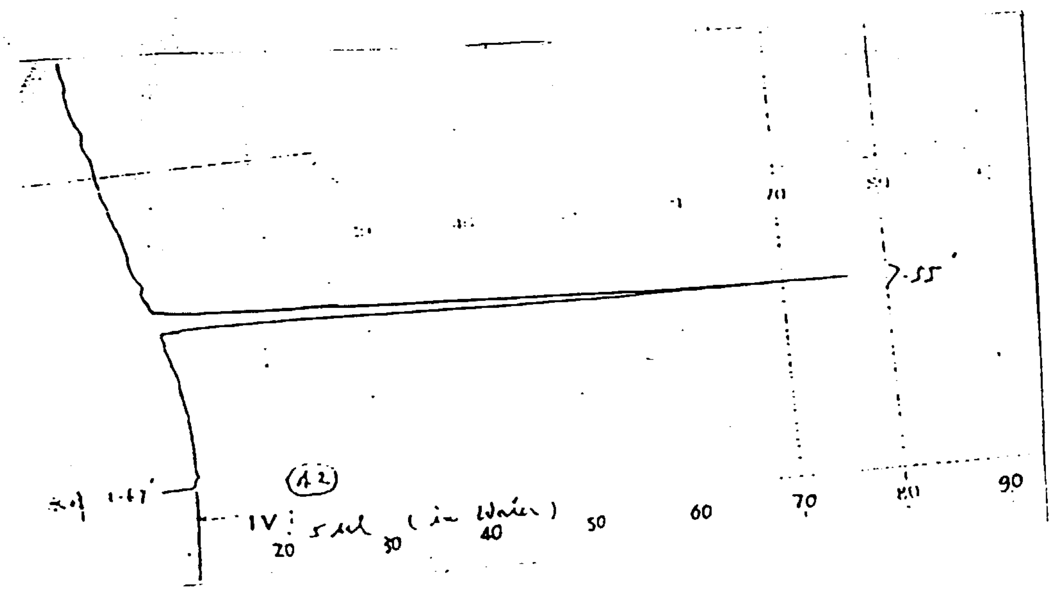

Figure 3: The HPLC spectra for the pure sample of LHRH antagonist V

Conditions:

Column: μ-Bondapak C18 (3,9 mm X 30 cm)

moving phase: A, 0.01 M $KH_2PO_4$ pH 3)

B, 20 % A + 80 % acetonitrile

gradient procedure: B from 40 % to 100 % in 15 minutes

flow rate: 2 ml/minute

detector: UV 210 nm

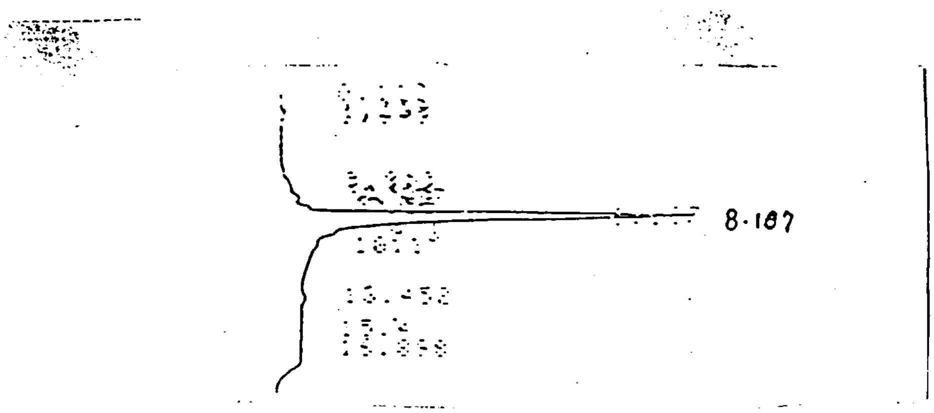

Figure 4: The HPLC spectra for the pure sample of LHRH antagonist VII

Conditions:

Column: μ-Bondapak C18 (3,9 mm X 30 cm)

moving phase: A, 0,01 M $KH_2PO_4$ (pH 3)

B, 20 % A + 80 % acetonitrile

gradient procedure: B from 40 % to 100 % in 15 minutes

flow rate: 1 ml/minute

detector: UV 210 nm

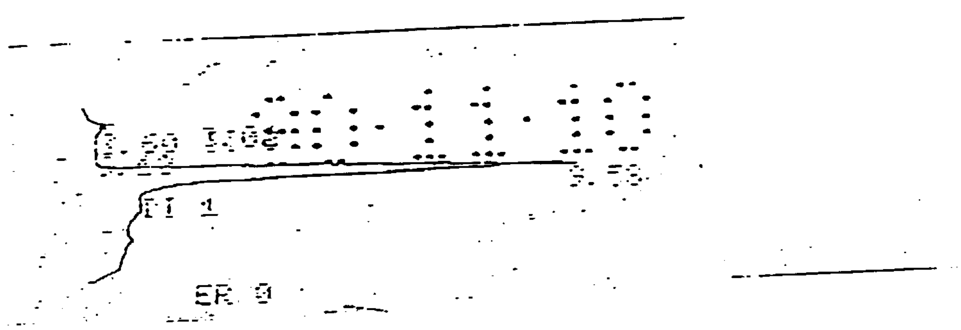

Figure 5: The PICO-TAGTM spectra of LHRH antagonist IV

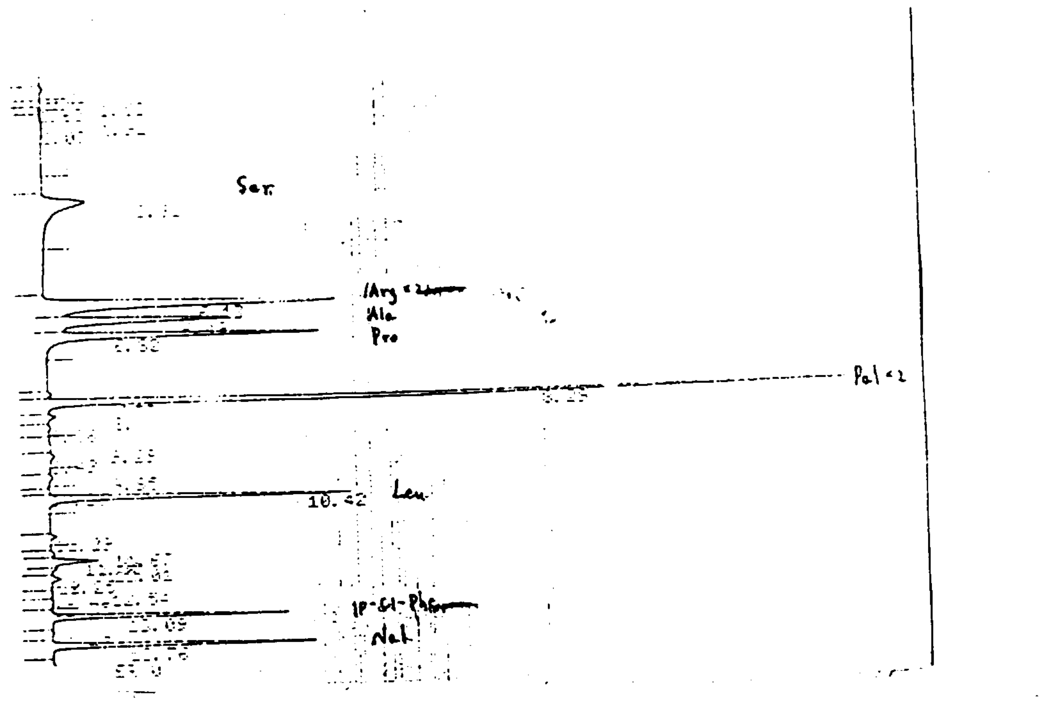

Figure 6: The PICO-TAGTM spectra of LHRH antagonist V